# FASCICULE DE BREVET EUROPEEN

(11) **EP 1 534 134 B1**
(45) Date de publication et mention de la délivrance du brevet: **24.10.2007**
(21) Numéro de dépôt: 03769535.0
(22) Date de dépôt: 29.08.2003
(51) Int. Cl.: A61B 5/22, A61B 5/103

(54) **PROCEDE ET DISPOSITIF DE MESURE DE FORCE MUSCULAIRE**
VERFAHREN UND VORRICHTUNG ZUR MESSUNG DER MUSKELKRAFT
MUSCLE STRENGTH MEASURING METHOD AND DEVICE

(30) Priorité: 02.09.2002 FR 0211082
(43) Date de publication de la demande: 01.06.2005
(73) Titulaire: Chatrenet, Yves, 74700 Sallanches (FR)
(72) Inventeur: Chatrenet, Yves, 74700 Sallanches (FR)
(74) Mandataire: Poncet, Jean-François
(86) Numéro de dépôt international: PCT/FR2003/002604
(87) Numéro de publication internationale: WO 2004/019781

(56) Documents cités:
- EP-A- 1 183 996
- FR-A- 2 661 600

## Description

### DOMAINE TECHNIQUE DE L'INVENTION

La présente invention concerne les appareils et procédés pour contrôler la capacité musculaire des muscles du dos, en particulier la capacité des muscles courts et profonds.

Le mal de dos est fréquemment associé à un manque de capacité musculaire des muscles assurant l'équilibrage de la colonne vertébrale.

On distingue à cet égard deux types de muscles : les muscles extenseurs, qui sont des muscles relativement longs et superficiels, utilisés pour les mouvements de grandes amplitudes, et les muscles courts et profonds, qui assurent en particulier les mouvements d'allongement de la colonne vertébrale.

Les muscles extenseurs ont déjà fait l'objet d'études particulières, et des dispositifs ont été conçus pour assurer leurs mesures et leurs contrôles.

Par contre, les muscles courts et profonds ont été négligés, et les appareils et procédés connus ne permettent pas de les contrôler.

### EXPOSE DE L'INVENTION

La présente invention vise essentiellement à contrôler la capacité musculaire des muscles courts et profonds du dos, en les faisant travailler en auto grandissement.

Les documents FR 2 661 600 et EP 1 183 996 décrivent des dispositifs de mesure de longueur du corps humain. De tels dispositifs sont destinés à mesurer la taille d'un patient, soit en position debout, soit en position couchée. Ces dispositifs ne permettent pas de contrôler de manière automatique la position du patient en cours de mesure de taille, ni de mesurer une force d'auto grandissement.

Les muscles courts et profonds du dos agissent pour le grandissement de la colonne vertébrale, et la mesure du grandissement dépend fortement de la position des pieds du patient : en position debout, le patient peut réaliser un faux grandissement en modifiant sa position plantaire normale, par exemple en se mettant sur la pointe des pieds (flexion plantaire), en soulevant l'avant-pied (flexion dorsale du pied), en fléchissant les orteils vers le bas, en roulant le pied vers l'extérieur (supination du pied). L'invention vise à éviter ou au moins à détecter un tel faux grandissement, de façon à mesurer le vrai grandissement de manière fiable et reproductible.

Ainsi, le problème principal proposé par l'invention est de concevoir des moyens pour évaluer de manière fiable et simple la capacité musculaire des muscles courts et profonds du dos.

De manière subsidiaire, le problème de l'invention est d'évaluer la capacité musculaire du psoas-illiaque dans des conditions assurant la fiabilité de ce contrôle. La difficulté est alors de mettre le patient dans une position reproductible permettant l'évaluation répétitive et fiable de la capacité musculaire du psoas-illiaque.

La fiabilité du contrôle nécessite en particulier que les segments corporels se retrouvent toujours dans une position définie lors du contrôle de capacité d'un muscle agissant sur ces segments.

L'invention profite alors de la position particulière que doit prendre le patient lors du contrôle des muscles courts et profonds, pour contrôler également de manière fiable le psoas-illiaque qui est le muscle permettant de lever la cuisse vers l'avant.

Pour atteindre ces objets ainsi que d'autres, l'invention propose un dispositif de mesure de force musculaire comprenant :
- une base inférieure d'appui, adaptée pour supporter un patient reposant debout en appui plantaire sur ladite base inférieure d'appui,
- une console supérieure d'appui, déplaçable verticalement au dessus de la base inférieure d'appui, conformée pour être en appui vertical sur la tête dudit patient,
- des moyens pour bloquer sélectivement la position verticale de la console supérieure d'appui,
- des moyens de mesure de la position verticale de la console supérieure d'appui,
- des moyens de mesure de la force verticale de soulèvement que la tête du patient applique sur la console supérieure d'appui,
- des capteurs d'appui plantaire prévus dans la base inférieure d'appui, et agencés pour contrôler le maintien d'un appui plantaire normal du ou des pieds du patient en produisant un signal si le ou les pieds ne sont plus en appui plantaire normal.

En appui plantaire normal, le patient tente de se grandir en faisant fonctionner les muscles courts et profonds du dos. Sa tête est en appui sous la console supérieure d'appui qui est elle-même soit bloquée en position verticale pour une mesure de force d'auto grandissement, soit libre en coulissement vertical pour une mesure d'amplitude d'auto grandissement. Le dispositif permet ainsi de mesurer les effets d'auto grandissement en amplitudes d'auto grandissement et en forces d'auto grandissement.

En pratique, la console supérieure d'appui peut être portée par un mât vertical la reliant à la base inférieure d'appui.

En alternative, la console supérieure d'appui peut être portée par un mur vertical près duquel repose la base inférieure d'appui.

Selon un mode de réalisation particulier, la base inférieure d'appui comprend des capteurs d'appui plantaire agencés pour s'assurer que la force d'appui est supérieure à un minimum prédéfini à la fois dans la zone antérieure et dans la zone postérieure du pied.

Selon un mode de réalisation particulier permettant le contrôle supplémentaire du psoas-illiaque, le dispositif comprend en outre un support antérieur, adapté pour constituer un appui frontal contre lequel peut porter la base antérieure de la cuisse du patient en fléchissant de moins de 30°, de préférence de moins de 20°, avec des moyens de mesure de force musculaire frontale pour évaluer la force d'appui frontal de la cuisse du patient.

Selon un mode de réalisation préféré, le dispositif comprend en outre une unité de calcul, associée à des moyens de mémoires et à des moyens d'affichage, et recevant les signaux provenant des moyens de mesure de position verticale et des capteurs d'appui plantaire de la base inférieure d'appui, les moyens de mémoires contenant un programme enregistré pour piloter l'unité de calcul, le programme enregistré contenant notamment une séquence de mesure d'amplitude d'auto grandissement par enregistrement des positions verticales de la console supérieure d'appui lorsque celle-ci est en coulissement libre et repoussée par la tête du patient, et une séquence de mesure des forces musculaires d'auto grandissement en enregistrant les valeurs de force de soulèvement appliquées par la tête du patient sur la console supérieure d'appui alors que ladite console supérieure d'appui est bloquée en une position verticale appropriée.

Le programme peut avantageusement comprendre une séquence de mesure d'endurance, par mesure du temps de maintien d'une force de soulèvement appropriée, appliquée par la tête du patient sur la console supérieure d'appui.

Selon un autre aspect, l'invention propose un procédé de mesure de force musculaire d'un patient, mettant en oeuvre le dispositif ci-dessus, et comprenant les étapes de :
a) installer le patient en position debout sur la base inférieure d'appui,
b) s'assurer en permanence que le patient est en appui plantaire normal sur la base inférieure d'appui, et interrompre les mesures si l'appui plantaire n'est pas normal,
c) mesurer l'amplitude d'auto grandissement du patient, en laissant coulisser la console supérieure d'appui selon les mouvements verticaux de la tête du patient, et en enregistrant les positions successives de la console supérieure d'appui,
d) déterminer la valeur maximale d'auto grandissement, correspondant à la position la plus haute enregistrée au cours de l'étape précédente,
e) fixer la position verticale de la console supérieure d'appui à une valeur inférieure de quelques millimètres à la valeur maximale d'auto grandissement,
f) mesurer les forces d'auto grandissement en enregistrant la force de soulèvement exercée par la tête du patient sur la console supérieure d'appui bloquée en position verticale.
   Selon un mode de réalisation avantageux, le procédé comprend en outre les étapes de :
g) enregistrer la force maximale de soulèvement,
h) choisir un seuil de force de soulèvement inférieur à la force maximale de soulèvement,
i) déterminer le temps maximal endurant (TME), par mesure du temps maximal de maintien d'une force de soulèvement supérieure ou égale au seuil de force de soulèvement.
   En alternative ou en complément, le procédé peut comprendre en outre les étapes de :
g) enregistrer la force maximale de soulèvement,
h) choisir un seuil de force de soulèvement inférieur à la force maximale de soulèvement,
j) piloter un travail par contractions intermittentes (TCI), en générant un signal intermittent décelable par le patient pour l'inciter à des alternances de contractions d'auto grandissement et de relâchements, en comptant le nombre de contractions atteignant le seuil de force de soulèvement après un relâchement suffisant caractérisé par une force de soulèvement suffisamment faible.
   Enfin, pour le contrôle du psoas-illiaque, le procédé peut avantageusement comprendre en outre les étapes de :
k) le patient étant en position de mesure de la force d'auto grandissement des étapes précédentes, mais en appui plantaire normal sur un seul pied et l'autre jambe fléchie, mesurer la force maximale d'appui frontal de la cuisse de jambe fléchie du patient contre un support antérieur,
l) choisir un seuil de force d'appui frontal inférieur à la force maximale d'appui frontal de la cuisse,
m) déterminer l'endurance du psoas-illiaque, en mesurant le temps maximal de maintien d'une force d'appui frontal supérieure ou égale au seuil de force d'appui frontal, et/ou en comptant le nombre de contractions alternées de périodes de relâchement et atteignant le seuil de force d'appui frontal.

### DESCRIPTION SOMMAIRE DES DESSINS

D'autres objets, caractéristiques et avantages de la présente invention ressortiront de la description suivante de modes de réalisation particuliers, faite en relation avec les figures jointes, parmi lesquelles :
- la figure 1 est une vue schématique de coté illustrant un patient en position de contrôle sur un dispositif de mesure de force musculaire selon un mode de réalisation de la présente invention ;
- la figure 2 est une vue de dessus de la base inférieure d'appui, illustrant les zones préférentielles de positionnement de capteurs d'appui pour le contrôle de l'appui plantaire ;
- la figure 3 est une vue schématique de coté illustrant un patient en position de contrôle de psoas-illiaque sur un dispositif selon un second mode de réalisation de la présente invention ;
- la figure 4 est un diagramme illustrant schématiquement les organes essentiels de mesure et de contrôle ainsi que leurs connections à une unité de calcul centrale ;
- la figure 5 illustre un mode de réalisation particulier des moyens de contrôle d'un appui plantaire normal du patient ; et
- la figure 6 illustre un autre mode de réalisation des moyens de contrôle d'un appui plantaire normal du patient.

### DESCRIPTION DES MODES DE REALISATION PREFERES

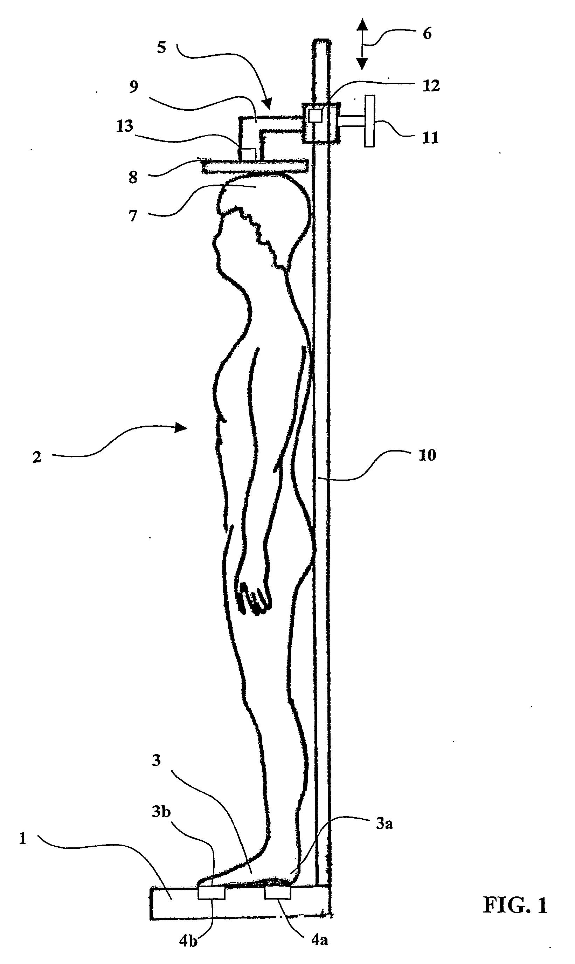

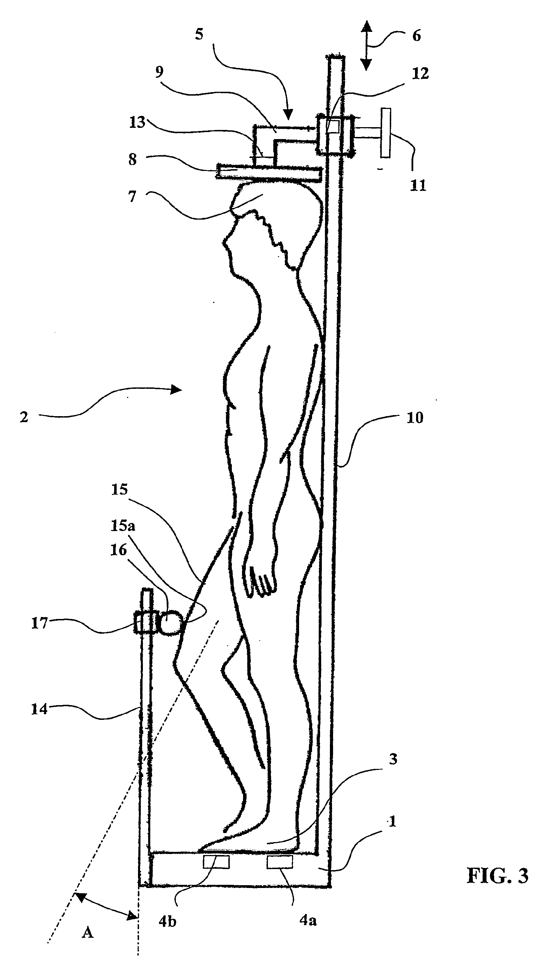

Dans le mode de réalisation illustré sur la figure 1, un dispositif de mesure de force musculaire selon l'invention comprend une base inférieure d'appui 1, adaptée pour supporter un patient 2 qui repose debout en appui plantaire par ses pieds 3 sur ladite base inférieure d'appui 1.

La base inférieure d'appui 1 est essentiellement un plateau horizontal, muni de moyens pour contrôler le maintien d'un appui plantaire normal du ou des pieds 3 du patient 2. Le but est de s'assurer que le pied 3 repose sur la base inférieure d'appui 1 par la plante du pied, sans effet de grandissement résultant par exemple du soulèvement du talon 3a par flexion plantaire, du soulèvement de la pointe de pied 3b par flexion dorsale du pied, du fléchissement des orteils et/ou de la supination du pied.

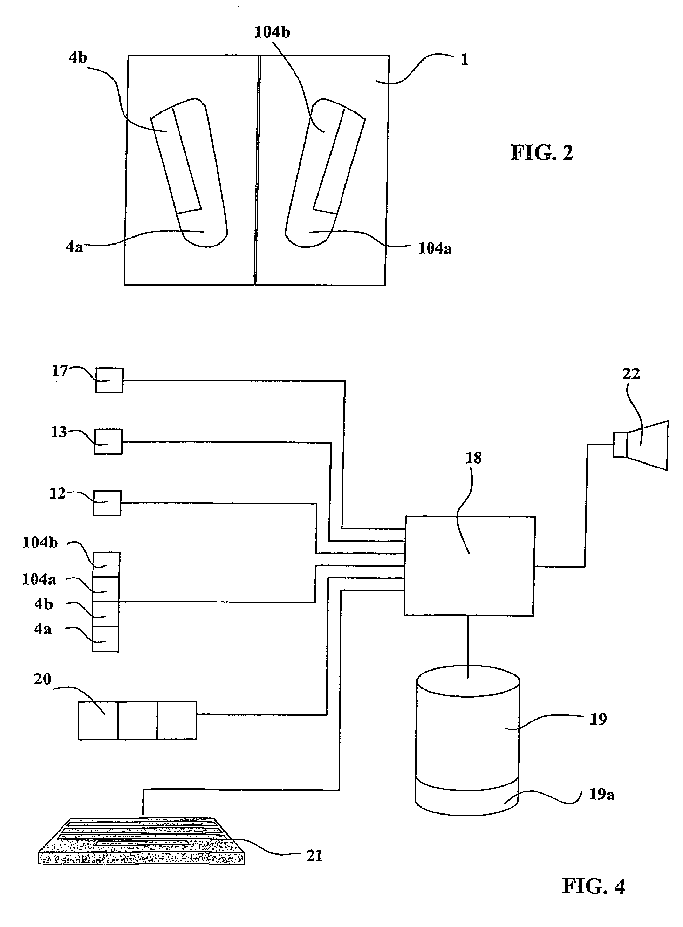

Selon un mode de réalisation simplifié, on peut par exemple placer des capteurs d'appui 4a sous la zone du talon 3a, et des capteurs d'appui 4b sous la zone de pointe de pied 3b, avec des moyens pour s'assurer que la force mesurée par les capteurs 4a et 4b ne devient pas inférieure à un seuil minimal prédéfini, par exemple un seuil égal à quelques Newtons. La figure 2 illustre une répartition possible des capteurs d'appui plantaire, avec les capteurs 4a et 4b ci-dessus pour un premier pied du patient, et avec des capteurs d'appui plantaire 104a et 104b pour l'autre pied du patient. D'autres modes de réalisation seront décrits plus loin.

Le dispositif comprend en outre une console supérieure d'appui 5, déplaçable verticalement comme indiqué par la flèche 6 au dessus de la base inférieure d'appui 1.

La console supérieure d'appui 5 est conformée pour être en appui vertical sur la tête 7 du patient 2, et comprend par exemple, pour cela, un plateau horizontal 8 tenu par un bras 9.

Dans la réalisation illustrée sur la figure 1, la console supérieure d'appui 5 est montée coulissante, selon l'extrémité postérieure du bras 9, le long d'un mât vertical 10 qui la relie à la base inférieure d'appui 1. Une vis de blocage 11 permet de bloquer sélectivement la console supérieure d'appui 5 en une position verticale choisie par l'opérateur.

La console supérieure d'appui 5 est associée à un capteur de position 12, permettant de mesurer la position verticale de la console supérieure d'appui 5.

Un ou des capteurs de force de soulèvement 13, placés dans la console supérieure d'appui 5 de façon appropriée, permettent de mesurer la force verticale de soulèvement appliquée par la tête 7 du patient 2 sur la console supérieure d'appui 5.

En alternative, les capteurs de force de soulèvement 13 peuvent être placés à l'interface entre le mât 10 et la console supérieure d'appui 5, ou en une zone intermédiaire de liaison entre la base inférieure d'appui 1 et la console supérieure d'appui 5, ou même entre le sol et la base inférieure d'appui 1. Dans ce dernier cas, la force de soulèvement est déterminée par différence entre le poids mesuré en l'absence d'effort de soulèvement et le poids mesuré en présence d'effort de soulèvement.

Dans le mode de réalisation de la figure 3, on retrouve les mêmes éléments que dans le mode de réalisation de la figure 1, et ces éléments sont repérés par les mêmes références numériques. Il n'est donc pas nécessaire de les décrire à nouveau.

Dans ce mode de réalisation de la figure 3, le dispositif comprend en outre un support antérieur 14, adapté pour constituer un appui frontal contre lequel peut porter la base antérieure 15a de la cuisse 15 du patient 2, dans une position dans laquelle la cuisse 15 fléchit selon un angle A de moins de 30°, et de préférence de moins de 20°.

Le support antérieur 14 comprend une barre transversale d'appui 16 et un capteur de force frontale 17 pour évaluer la force d'appui frontal de la cuisse 15 du patient 2.

De préférence, la barre transversale d'appui 16 est réglée en hauteur par coulissement et blocage le long du support antérieur 14, pour son adaptation à la morphologie du patient 2.

On se référera maintenant à la figure 4, sur laquelle on retrouve les capteurs d'appui plantaire 4a et 4b, le capteur de position 12, le capteur de force de soulèvement 13, le capteur de force frontale 17, ainsi que les capteurs d'appui plantaire similaires 104a et 104b pour l'autre pied du patient.

Tous ces capteurs produisent sur leurs sorties des signaux envoyés par des lignes correspondantes à une unité de calcul 18 telle qu'un micro contrôleur ou un micro processeur. L'unité de calcul 18 est associée à des moyens de mémoires 19, à des moyens d'affichage de sortie 20, et de préférence à des moyens d'entrée de données tels qu'un clavier 21. De préférence l'unité de calcul 18 est également reliée à un générateur de signal 22 perceptible par le patient 2, par exemple un générateur de signal audible ou de signal lumineux. Dans la mémoire 19 se trouve une zone de programme 19a ayant un programme enregistré.

Le programme enregistré dans la zone de programme 19a contient notamment une séquence de mesure d'auto grandissement par enregistrement des positions verticales successives de la console supérieure d'appui 5 en coulissement libre repoussé par la tête 7 du patient 2, et une séquence de mesure des forces musculaires maximales d'auto grandissement par enregistrement des valeurs de force de soulèvement appliquées par la tête 7 du patient 2 sur la console supérieure d'appui 5 alors que ladite console supérieure d'appui 5 est bloquée par la vis 11 en une position verticale appropriée.

Le programme peut avantageusement comprendre une séquence de mesure d'endurance, par mesure de temps de maintien d'une force de soulèvement appropriée appliquée par la tête 7 du patient 2 sur la console supérieure d'appui 5.

Au cours de la séquence de mesure d'auto grandissement, l'unité de calcul 18 scrute les signaux produits par le capteur de position 12, enregistre dans la mémoire 19 les positions verticales successives de la console supérieure d'appui 5, et détermine la valeur maximale du soulèvement ou valeur maximale d'auto grandissement.

L'opérateur bloque alors la console supérieure d'appui 5 en une position verticale située à quelques millimètres en dessous de la valeur maximale d'auto grandissement, par serrage de la vis 11. Au cours de la séquence de mesure des forces musculaires d'auto grandissement, qui fait suite à la séquence de mesure d'auto grandissement précédente, l'unité de calcul 18 scrute les signaux provenant du capteur de force de soulèvement 13, enregistre dans la mémoire 19 la suite des valeurs de force de soulèvement appliquées par la tête 7 du patient 2 sur la console supérieure d'appui 5. Le programme détermine ensuite la valeur maximale de la force de soulèvement.

On choisit ensuite un seuil de force de soulèvement inférieur à la force maximale de soulèvement, soit de façon automatique par le programme enregistré dans la zone de programme 19a, soit de façon manuelle par intervention de l'opérateur sur le clavier 21.

Au cours de la séquence suivante de mesure d'endurance, l'unité de calcul 18 scrute les signaux provenant du capteur de force de soulèvement 13, et mesure le temps maximal de maintien d'une force de soulèvement supérieure ou égale au seuil de force de soulèvement précédemment défini. On détermine ainsi le temps maximal endurant (TME). On cesse la mesure d'endurance lorsque la force de soulèvement est au dessous du seuil de force de soulèvement pendant une durée supérieure à trois secondes.

Le dispositif permet également de piloter un travail par contractions intermittentes (TCI). Dans ce cas, le programme enregistré contient des séquences d'instructions utilisées par l'unité de calcul 18 qui pilote alors le générateur de signal 22 pour générer ainsi un signal intermittent décelable par le patient 2 pour l'inciter à des alternances de contractions d'auto grandissement et de relâchement. L'unité de calcul scrute les signaux provenant du capteur de force de soulèvement 13, et compte le nombre de contractions atteignant le seuil de force de soulèvement après un relâchement suffisant caractérisé par une force de soulèvement suffisamment faible détectée par le capteur de force de soulèvement 13.

Dans le cas du fonctionnement d'un dispositif de mesure de force musculaire du psoas-illiaque tel que défini sur la figure 3, l'unité de calcul 18 scrute les signaux provenant du capteur de force frontale 17, et mesure les forces d'appui de la cuisse 15 du patient 2 contre le support antérieur 14. L'unité de calcul 18 détermine ensuite la force d'appui frontal maximale enregistrée dans la mémoire 19, puis on choisit un seuil de force d'appui frontal qui soit inférieur à la force d'appui frontal maximale enregistrée.

Le programme enregistré dans la zone de programme 19a contient ensuite une séquence de détermination d'endurance du psoas-illiaque, au cours de laquelle l'unité de calcul 18 scrute les signaux provenant du capteur de force frontale 17, mesure le temps maximal de maintien d'une force d'appui frontal supérieure ou égale au seuil de force d'appui frontal, et/ou en comptant le nombre de contractions alternées de périodes de relâchement et atteignant le seuil de force d'appui frontal.

Dans la mise en oeuvre du dispositif défini ci-dessus, l'invention prévoit un procédé de mesure de force musculaire d'un patient comprenant une suite d'étapes au cours desquelles :
a) on installe le patient 2 en position debout avec ses deux pieds sur la base inférieure d'appui 1,
b) on s'assure en permanence que le patient 2 est en appui plantaire normal sur la base inférieure d'appui 1, c'est à dire en appui suffisant à la fois sur ses pointes de pied 3b et sur ses talons 3a, et on interrompt les mesures si l'appui plantaire n'est pas normal,
c) on adapte la console supérieure d'appui sur la tête 7 du patient 2, et on mesure l'auto grandissement du patient 2 en laissant coulisser la console supérieure d'appui 5 selon les mouvements verticaux de la tête 7 du patient 2, et on enregistre les positions successives de la console supérieure d'appui 5,
d) on détermine la valeur maximale d'auto grandissement, correspondant à la position la plus haute enregistrée au cours de l'étape précédente,
e) on fixe la position verticale de la console supérieure d'appui 5 à une valeur inférieure de quelques millimètres, par exemple 5 mm, à la valeur maximale d'auto grandissement,
f) on mesure les forces d'auto grandissement en enregistrant la force de soulèvement exercée par la tête 7 du patient 2 sur la console supérieure d'appui 5.
   Pour déterminer le temps maximal endurant (TME), on poursuit par les étapes suivantes :
g) on enregistre la force maximale de soulèvement,
h) on choisit un seuil de force de soulèvement inférieur à la force maximale de soulèvement,
i) on détermine le temps maximal endurant par la mesure du temps maximal de maintien d'une force de soulèvement supérieure ou égale au seuil de force de soulèvement.
   Pour piloter un travail par contractions intermittentes (TCI), le procédé comprend les étapes suivantes :
g) on enregistre la force maximale de soulèvement,
h) on choisit un seuil de force de soulèvement inférieur à la force maximale de soulèvement,
j) on pilote le travail par contractions intermittentes (TCI) en générant un signal intermittent décelable par le patient pour l'inciter à des alternances de contractions d'auto grandissement et de relâchements, en comptant le nombre de contractions atteignant le seuil de force de soulèvement après un relâchement suffisant caractérisé par une force de soulèvement suffisamment faible.
   Pour le contrôle du psoas-illiaque, le procédé selon l'invention comprend en outre les étapes suivantes :
k) le patient étant en position de mesure de force d'auto grandissement telle que définie ci dessus, mais en appui plantaire normal sur un seul pied, l'autre jambe étant fléchie pour venir porter frontalement par la base de cuisse 15a sur une barre transversale d'appui 16, on mesure la force maximale d'appui frontal de la cuisse 15 du patient 2 contre le support antérieur 14,
l) on choisit un seuil de force d'appui frontal inférieur à la force maximale d'appui frontal précédemment déterminée de la cuisse 15,
m) on détermine l'endurance du psoas-illiaque en mesurant le temps maximal de maintien d'une force d'appui frontal supérieure ou égale au seuil de force d'appui frontal, et/ou en comptant le nombre de contractions alternées de périodes de relâchement et atteignant le seuil de force d'appui frontal.

Lors du contrôle des muscles courts et profonds par auto grandissement, l'efficacité de l'invention résulte d'un contrôle permanent de la position du patient 2, et en particulier de son appui plantaire normal sur deux pieds.

Certains mouvements de faux grandissement peuvent être détectés par le mode de réalisation simplifié illustré en relation avec les figures 1 et 3.

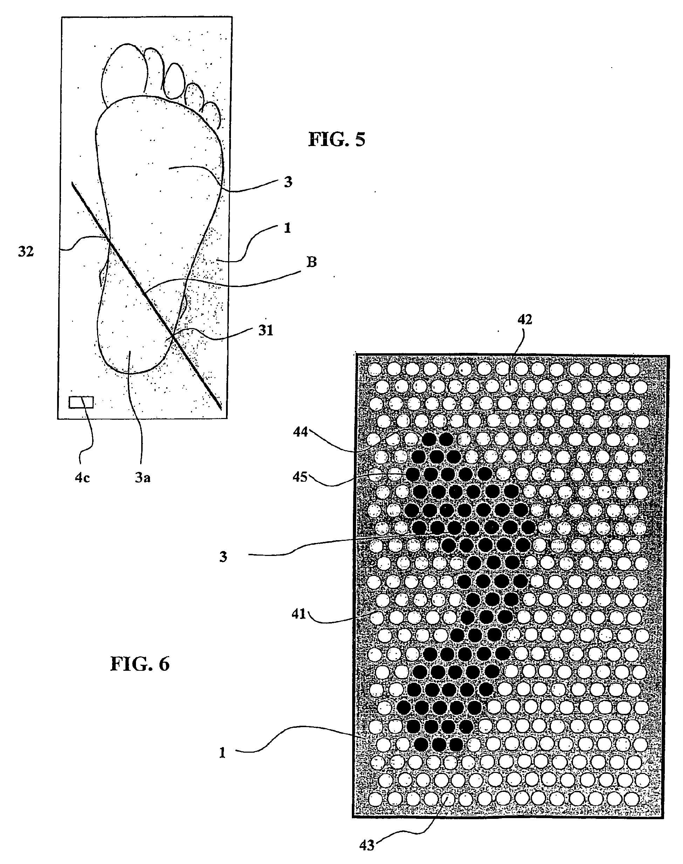

Pour détecter un plus grand nombre de mouvements de faux auto grandissement, on peut utiliser des moyens perfectionnés qui sont décrits ci-après en relation avec les figures 5 et 6.

Comme illustré sur la figure 5, chaque pied repose sur une base inférieure d'appui 1 sous laquelle un axe de bascule B oblique est intégré. L'axe de bascule B est orienté obliquement selon un axe d'environ 30° à 60°, de telle sorte que le pied 3 soit positionné au dessus de l'axe B, avec l'axe B croisant le bord postéro externe 31 du talon 3a et croisant une zone intermédiaire 32 du bord interne du pied 3. On prévoit un capteur de contact ou de proximité 4c sous la partie postérieure et interne de la base inférieure d'appui 1. Toute tentative de mise en flexion plantaire et/ou de supination du pied entraîne une bascule de la base inférieure d'appui 1 et une perte de contact détectée par le capteur de contact ou de proximité 4c, qui inhibe alors la mesure d'auto grandissement.

Selon un mode de réalisation perfectionné, illustré sur la figure 6, chaque plate-forme d'appui telle que la base inférieure d'appui 1 comprend une matrice de capteurs ou contacteurs tels que les capteurs ou contacteurs 41, 42, 43, 44 ou 45 répartis sur la surface de base inférieure d'appui 1 destinée à recevoir l'appui du pied 3. Chaque capteur ou contacteur 41-45 peut avoir une dimension d'environ 1 cm², et être de nature mécanique, électrique, thermique, photosensible, capacitive ou autre. Chaque capteur 41-45 est connecté à l'unité de calcul 18 (figure 4), et produit un signal de mesure de pression qui est analysé par l'unité de calcul. Le programme enregistré dans la mémoire 19a pilote l'unité de calcul 18 pour scruter périodiquement chaque capteur 41-45. En début de mesure, l'unité de calcul 18 peut ainsi déterminer la zone d'appui du pied 3, ou zone figurée en noir sur la figure 6 correspondant à l'ensemble des capteurs recevant une pression du pied. La perte de contact ou de pression ultérieure d'un ou de plusieurs capteurs activés initialement par l'appui plantaire normal génère le non fonctionnement du système de mesure des forces d'auto grandissement.

Les principaux capteurs sont positionnés sous la partie inférieure et interne du pied d'une part, et sous le talon d'autre part.

Selon une possibilité, le programme peut déterminer la résultante de l'ensemble des pressions reçues par les capteurs de chaque pied, et variant au delà d'une tolérance définie par l'opérateur du système. Toute modification de l'appui plantaire fait varier cette résultante, et sa variation au delà d'une tolérance définie par l'opérateur du système entraîne l'inhibition du système de mesure des forces d'auto grandissement.

Un capteur 44 placé sous la seconde phalange du gros orteil, et un capteur 45 placé sous la tête du premier méta tarsien permettent de détecter respectivement l'extension du gros orteil, ou l'ascension de la tête du premier méta tarsien lors de la flexion des orteils vers le bas, et détectent également la flexion dorsale du pied. La désactivation de l'un ou l'autre des deux capteurs 44 et 45 entraîne l'inhibition du système de mesure des forces d'auto grandissement.

On comprend que le dispositif illustré sur la figure 6 permet de détecter tous les mouvements de faux grandissement.

On peut naturellement imaginer des dispositifs intermédiaires, permettant de détecter certains seulement des mouvements de faux grandissement. Par exemple, on peut placer un contacteur ou capteur dont le déplacement est effectué par glissement sur le bord interne de chaque plate-forme et positionné au dessus du premier orteil. La mise en extension du gros orteil active ce contacteur et entraîne l'inhibition du système de mesure des forces d'auto grandissement.

Lors du contrôle du psoas-illiaque, la précision de l'invention résulte également du contrôle permanent de l'auto grandissement du patient 2, en particulier de la position de son bassin d'où résulte notamment son grandissement.

La présente invention n'est pas limitée aux modes de réalisation qui ont été explicitement décrits, mais elle en inclut les diverses variantes et généralisations contenues dans le domaine des revendications ci-après.

## Revendications

1. - Dispositif de mesure de force musculaire comprenant:
- une base inférieure d'appui (1), adaptée pour supporter un patient (2) reposant debout en appui plantaire sur ladite base inférieure d'appui (1),
- une console supérieure d'appui (5), déplaçable verticalement au dessus de la base inférieure d'appui (1), conformée pour être en appui vertical sur la tête (7) dudit patient (2),
- des moyens (11) pour bloquer sélectivement la position verticale de la console supérieure d'appui (5),
- des moyens de mesure (12) de la position verticale de la console supérieure d'appui (5),
- des moyens de mesure (13) de la force verticale de soulèvement que la tête (7) du patient (2) applique sur la console supérieure d'appui (5),
- des capteurs d'appui plantaire (4a, 4b, 104a, 104b), prévus dans la base inférieure d'appui (1), et agencés pour contrôler le maintien d'un appui plantaire normal du ou des pieds (3) du patient (2) en produisant un signal si le ou les pieds (3) ne sont plus en appui plantaire normal.

2. - Dispositif selon la revendication 1, **caractérisé en ce que** la console supérieure d'appui (5) est portée par un mât vertical (10) la reliant à la base inférieure d'appui (1).

3. - Dispositif selon l'une des revendications 1 ou 2, **caractérisé en ce que** la base inférieure d'appui (1) comprend des capteurs d'appui plantaire (4a, 4b, 104a, 104b) agencés, pour s'assurer que la force d'appui est supérieure à un minimum prédéfini à la fois dans la zone antérieure (3b) et dans la zone postérieure (3a) du pied (3).

4. - Dispositif selon l'une quelconque des revendications 1 à 3, **caractérisé en ce qu'**il comprend en outre un support antérieur (14), adapté pour constituer un appui frontal contre lequel peut porter la base antérieure (15a) de la cuisse (15) du patient (2) en fléchissant de moins de 30°, de préférence de moins de 20°, avec des moyens de mesure (17) de force musculaire frontale pour évaluer la force d'appui frontal de la cuisse (15) du patient (2).

5. - Dispositif selon l'une quelconque des revendications 1 à 4, **caractérisé en ce qu'**il comprend une unité de calcul (18), associée à des moyens de mémoires (19) et à des moyens d'affichage (20), et recevant les signaux provenant des moyens de mesure de position verticale (12) et des capteurs d'appui plantaire (4a, 4b, 104a, 104b) de la base inférieure d'appui (1), les moyens de mémoires (19) contenant un programme enregistré pour piloter l'unité de calcul (18), le programme enregistré contenant notamment une séquence de mesure d'amplitude d'auto grandissement par enregistrement des positions verticales de la console supérieure d'appui (5) lorsque celle-ci est en coulissement libre et repoussée par la tête (7) du patient (2), et une séquence de mesure des forces musculaires d'auto grandissement en enregistrant les valeurs de force de soulèvement appliquées par la tête (7) du patient (2) sur la console supérieure d'appui (5) alors que ladite console supérieure d'appui (5) est bloquée en une position verticale appropriée.

6. - Dispositif selon la revendication 5, **caractérisé en ce que** le programme enregistré comprend une séquence de mesure d'endurance, par mesure du temps de maintien d'une force de soulèvement appropriée appliquée par la tête (7) du patient (2) sur la console supérieure d'appui (5).

7. - Dispositif selon l'une des revendications 5 ou 6, **caractérisé en ce que** le programme enregistré comprend des séquences d'instructions pour piloter un travail par contractions intermittentes (TCI), en générant un signal intermittent décelable par le patient (2) pour l'inciter à des alternances de contractions d'auto grandissement et de relâchements, en comptant le nombre de contractions atteignant le seuil de force de soulèvement après un relâchement suffisant **caractérisé par** une force de soulèvement suffisamment faible.

8. - Procédé de mesure de force musculaire d'un patient (2) mettant en oeuvre un dispositif selon l'une quelconque des revendications 1 à 7, **caractérisé en ce qu'**il comprend les étapes de :
a) installer le patient (2) en position debout sur la base inférieure d'appui (1),
b) s'assurer en permanence que le patient (2) est en appui plantaire normal sur la base inférieure d'appui (1), et interrompre les mesures si l'appui plantaire n'est pas normal,
c) mesurer l'amplitude d'auto grandissement du patient (2), en laissant coulisser la console supérieure d'appui (5) selon les mouvements verticaux de la tête (7) du patient (2), et en enregistrant les positions successives de la console supérieure d'appui (5),
d) déterminer la valeur maximale d'auto grandissement, correspondant à la position la plus haute enregistrée au cours de l'étape précédente,
e) fixer la position verticale de la console supérieure d'appui (5) à une valeur inférieure de quelques millimètres à la valeur maximale d'auto grandissement,
f) mesurer les forces d'auto grandissement en enregistrant la force de soulèvement exercée par la tête (7) du patient (2) sur la console supérieure d'appui (5) bloquée en position verticale.

9. - Procédé selon la revendication 8, **caractérisé en ce qu'**il comprend en outre les étapes de :
g) enregistrer la force maximale de soulèvement,
h) choisir un seuil de force de soulèvement inférieur à la force maximale de soulèvement,
i) déterminer le temps maximal endurant (TME), par mesure du temps maximal de maintien d'une force de soulèvement supérieure ou égale au seuil de force de soulèvement.

10. - Procédé selon la revendication 8, **caractérisé en ce qu'**il comprend en outre les étapes de :
g) enregistrer la force maximale de soulèvement,
h) choisir un seuil de force de soulèvement inférieur à la force maximale de soulèvement,
j) piloter un travail par contractions intermittentes (TCI), en générant un signal intermittent décelable par le patient (2) pour l'inciter à des alternances de contractions d'auto grandissement et de relâchements, en comptant le nombre de contractions atteignant le seuil de force de soulèvement après un relâchement suffisant **caractérisé par** une force de soulèvement suffisamment faible.

11. - Procédé selon l'une quelconque des revendications 8 à 10, **caractérisé en ce qu'**il comprend en outre les étapes de :
k) le patient (2) étant en position de mesure de la force d'auto grandissement des étapes précédentes, en appui plantaire normal sur un pied (3), mesurer la force maximale d'appui frontal de la cuisse (15) du patient (2) contre le support antérieur (14),
l) choisir un seuil de force d'appui frontal inférieur à la force maximale d'appui frontal de la cuisse (15),
m) déterminer l'endurance du psoas-illiaque, en mesurant le temps maximal de maintien d'une force d'appui frontal supérieure ou égale au seuil de force d'appui frontal, et/ou en comptant le nombre de contractions alternées de périodes de relâchement et atteignant le seuil de force d'appui frontal.

## Claims

1. Device for measuring muscle strength, comprizing :
- a lower support base (1), adapted to support a standing patient (2) in plantar support on said lower support base (1),
- an upper support bracket (5), movable vertically above the lower support base (1), conformed to bear vertically on the head (7) of said patient (2),
- means (11) for selectively immobilizing the upper bearing bracket (5) in vertical position,
- means (12) for measuring the vertical position of the upper support bracket (5),
- means (13) for measuring the vertical lifting force that the head (7) of the patient (2) applies to the upper support bracket (5),
- plantar support sensors (4a, 4b, 104a, 104b), provided in the lower support base (1), and adapted to test for maintained normal plantar support of the foot or feet (3) of the patient (2) by producing a signal if the foot or feet (3) are no longer in normal plantar support.

2. Device according to claim 1, **characterized in that** the upper support bracket (5) is carried by a vertical column (10) connecting it to the lower support base (1).

3. Device according to either claim 1 or claim 2, **characterized in that** the lower support base (1) comprises plantar support sensors (4a, 4b, 104a, 104b) adapted to ensure that the bearing force is greater than a predefined minimum in both the anterior area (3b) and the posterior area (3a) of the foot (3).

4. Device according to any one of claims 1 to 3, **characterized in that** it further comprises an anterior support (14) adapted to constitute a frontal bearing against which the anterior base (15a) of the thigh (15) of the patient (2) can bear on flexing by less than 30°, preferably less than 20°, with means (17) for measuring the forward muscular force to evaluate the frontal bearing force of the thigh (15) of the patient (2).

5. Device according to any one of claims 1 to 4, **characterized in that** it comprises a computation unit (18), associated with memory means (19) and display means (20), and receiving signals from the vertical position measuring means (12) and the plantar support sensors (4a, 4b, 104a, 104b) of the lower bearing base (1), the memory means (19) containing a stored program for controlling the computation unit (18), the stored program including in particular a self-stretching amplitude measuring sequence for storing vertical positions of the upper support bracket (5) when the latter is allowed to slide freely and is pushed by the head (7) of the patient (2) and a self-stretching muscular force measuring sequence for storing values of the lifting force applied by the head (7) of the patient (2) to the upper support bracket (5) when said upper support bracket (5) is immobilized in an appropriate vertical position.

6. Device according to claim 5, **characterized in that** the stored program comprizes an endurance measurement sequence for measuring the time for which an appropriate lifting force applied by the head (7) of the patient (2) to the upper support bracket (5) is maintained.

7. Device according to either claim 5 or claim 6, **characterized in that** the stored program includes sequences of instructions for controlling a work by intermittent contraction (TCI) mode, by generating an intermittent signal detectable by the patient (2) to prompt alternate self-stretching contractions and relaxations, and counting the number of contractions reaching a lifting force threshold after a sufficient relaxation **characterized by** a sufficiently low lifting force.

8. Method of measuring the muscular strength of a patient (2) using a device according to any one of claims 1 to 7, **characterized in that** it comprises the steps of :
a) placing the patient (2) in a standing position on the lower support base (1),
b) ensuring continuously that the patient (2) is in normal plantar support on the lower bearing base (1), and interrupting the measurement if the plantar support is not normal,
c) measuring the amplitude of self-stretching of the patient (2), by allowing the upper support bracket (5) to slide up and down according to vertical movements of the head (7) of the patient (2), and storing successive positions of the upper support bracket (5),
d) determining the maximum self-stretching value corresponding to the highest position recorded during the previous step,
e) fixing the vertical position of the upper support bracket (5) a few millimeters below the maximum self-stretching value,
f) measuring the self-stretching forces by storing the lifting force exerted by the head (7) of the patient (2) on the upper support bracket (5) when the latter is immobilized vertically.

9. Method according to claim 8, **characterized in that** it further comprises the steps of :
g) recording the maximum lifting force,
h) selecting a lifting force threshold lower than the maximum lifting force,
i) determining the maximum endurance time (TME) by measuring the maximum time for which a lifting force greater than or equal to the lifting force threshold is maintained.

10. Method according to claim 8, **characterized in that** it further comprises the steps of :
g) storing the maximum lifting force,
h) selecting a lifting force threshold below the maximum lifting force,
j) in an intermittent contraction (TCI) mode, generating an intermittent signal detectable by the patient (2) to prompt alternate self-stretching contractions and relaxations and counting the number of contractions reaching a lifting force threshold after a sufficient relaxation **characterized by** a sufficiently low lifting force.

11. Method according to any one of claims 8 to 10, **characterized in that** it further comprises the steps of :
k) with the patient (2) in the position of the preceding steps for measuring the self-stretching force, in normal plantar support on one foot (3), measuring the maximum frontal force with which the thigh (15) of the patient (2) bears against the anterior support (14),
l) selecting a frontal bearing force threshold less than the maximum frontal bearing force exerted by the thigh (15),
m) determining the endurance of the psoas major muscle by measuring the maximum time for which a frontal bearing force greater than or equal to the frontal bearing force threshold is maintained and/or by counting the number of alternating contractions in periods of relaxation and reaching the frontal bearing force threshold.

## Patentansprüche

1. Einrichtung zur Messung der Muskelkraft enthaltend:
- eine untere Abstützbasis (1), die zum Abstützen eines Patienten (2) ausgebildet ist, der auf der genannten unteren Abstützbasis (1) mit Fußsohlenauflage steht,
- eine obere Abstützkonsole (5), die vertikal oberhalb der unteren Abstützbasis (1) verschieblich ist und zur vertikalen Anlage auf dem Kopf (7) des genannten Patienten (2) angepaßt ist,
- Mittel (11) zum wahlweisen Blockieren der vertikalen Position der oberen Abstützkonsole (5),
- Mittel (12) zum Messen der vertikalen Position der oberen Abstützkonsole (5),
- Mittel (13) zum Messen der vertikalen Hebekraft, die der Kopf (7) des Patienten (2) auf die obere Abstützkonsole (5) aufbringt,
- Fußsohlenauflagesensoren (4a, 4b, 104a, 104b), die in der unteren Abstützbasis (1) vorgesehen sind und zum Überwachen der Aufrechterhaltung einer normalen Fußsohlenauflage des Fußes oder der Füße (3) des Patienten (2) ausgebildet sind, indem sie ein Signal erzeugen, wenn der Fuß oder die Füße (3) nicht mehr in normaler Fußsohlenauflage sind.

2. Einrichtung nach Anspruch 1, **dadurch gekennzeichnet, daß** die obere Abstützkonsole (5) an einem vertikalen Ständer (10) getragen ist, der sie mit der unteren Abstützbasis (1) verbindet.

3. Einrichtung nach einem der Ansprüche 1 oder 2, **dadurch gekennzeichnet, daß** die untere Abstützbasis (1) Fußsohlenauflagesensoren (4a, 4b, 104a, 104b) enthält, die angepaßt sind, um sicherzustellen, daß die Auflagekraft größer ist als ein vordefiniertes Minimum sowohl in der vorderen Zone (3b) als auch in der hinteren Zone (3a) des Fußes (3).

4. Einrichtung nach irgendeinem der Ansprüche 1 bis 3, **dadurch gekennzeichnet, daß** sie weiterhin eine vordere Abstützung (14) enthält, die ausgebildet ist, um einen vorderen Anschlag zu bilden, gegen den die Vorderseite (15a) des Oberschenkels (15) des Patienten (2) unter Abbiegung von mindestens 30°, vorzugsweise mindestens 20°, anliegen kann, mit Mitteln (17) zum Messen der vorwärts gerichteten Muskelkraft zur Auswertung der vorwärtsgerichteten Anlagekraft des Oberschenkels (15) des Patienten (2).

5. Einrichtung nach irgendeinem der Ansprüche 1 bis 4, **dadurch gekennzeichnet, daß** sie eine Recheneinheit (18) enthält, der Speichermittel (19) und Anzeigemittel (20) zugeordnet sind, und die Signale empfängt, die von den Mitteln (12) zum Messen der vertikalen Position und den Fußsohlenauflagesensoren (4a, 4b, 104a, 104b) der unteren Abstützbasis (1) stammen, wobei die Speichermittel (19) ein gespeichertes Programm enthalten, um die Recheneinheit (18) zu steuern, wobei das gespeicherte Programm insbesondere eine Meßsequenz der Amplitude der Selbstvergrößerung enthält zum Speichern der vertikalen Position der oberen Abstützkonsole (5), wenn diese in freier Verschiebung ist und gegen den Kopf (7) des Patienten (2) gedrückt wird, und eine Meßsequenz der selbst-vergrößernden Muskelkräfte, in der Werte der angelegten Hebekraft, die von dem Kopf (7) des Patienten (2) auf die obere Abstützkonsole (5) ausgeübt werden, gespeichert werden, wenn die genannte obere Abstützkonsole (5) in einer entsprechenden vertikalen Position blockiert ist.

6. Einrichtung nach Anspruch 5, **dadurch gekennzeichnet, daß** das gespeicherte Programm eine Ausdauermeßsequenz enthält zum Messen der Zeit des Aufrechterhaltens einer entsprechenden Hebekraft, die von dem Kopf (7) des Patienten (2) auf die obere Abstützkonsole (5) aufgebracht wird.

7. Einrichtung nach einem der Ansprüche 5 oder 6, **dadurch gekennzeichnet, daß** das gespeicherte Programm eine Instruktionssequenz enthält zum Steuern einer intermittierenden Kontraktionsarbeit (TCI), indem ein intermittierendes Signal erzeugt wird, das von dem Patienten (2) erkannt wird, um mit Alternierungen von selbst-vergrößernden Kontraktionen und Entspannungen anzusprechen, indem man die Anzahl der Kontraktionen zählt, die eine Hebekraftschwelle nach einer ausreichenden Entspannung erreichen, die durch eine ausreichend niedrige Hebekraft **gekennzeichnet** sind.

8. Verfahren zum Messen der Muskelkraft eines Patienten (2) unter Verwendung einer Einrichtung nach irgendeinem der Ansprüche 1 bis 7, **dadurch gekennzeichnet, daß** es folgende Schritte aufweist:
a) Stellen eines Patienten (2) in aufrechter Position auf die untere Abstützbasis (1),
b) ständig Sicherstellen, daß der Patient (2) in normaler Fußsohlenauflage auf der unteren Abstützbasis (1) steht und Unterbrechen der Messungen, wenn die Fußsohlenauflage nicht normal ist,
c) Messen der Amplitude der Selbstvergrößerung des Patienten (2), indem man die obere Abstützkonsole (5) mit vertikalen Bewegungen des Kopfes (7) des Patienten (2) gleiten läßt und die aufeinanderfolgenden Positionen der oberen Abstützkonsole (5) registriert,
d) Bestimmen des maximalen Wertes der Selbstvergrößerung entsprechend der höchsten aufgezeichneten Position während des vorhergehenden Schrittes,
e) Fixieren der vertikalen Position der oberen Abstützkonsole (5) bei einem Wert einige Millimeter unterhalb des maximalen Wertes der Selbstvergrößerung,
f) Messen der Kräfte der Selbstvergrößerung durch Registrieren der von dem Kopf (7) des Patienten (2) auf die obere Abstützkonsole (5), die in vertikaler Position blockiert ist, aufgebracht werden.

9. Verfahren nach Anspruch 8, **dadurch gekennzeichnet, daß** es weiterhin folgende Schritte enthält:
g) Registrieren der maximalen Hebekraft,
h) Wählen eines Schwellwertes der Hebekraft unterhalb der maximalen Hebekraft,
i) Bestimmen der maximalen Ausdauerzeit (TME) durch Messen der maximalen Aufrechterhaltungszeit, für die eine Hebekraft größer oder gleich eines Hebekraftschwellwertes.

10. Verfahren nach Anspruch 8, **dadurch gekennzeichnet, daß** es weiterhin folgende Schritte enthält:
g) Registrieren der maximalen Hebekraft,
h) Wählen eines Schwellwertes der Hebekraft unterhalb der maximalen Hebekraft,
j) Steuern einer Arbeit mit intermittierenden Kontraktionen (TCI) durch Erzeugen eines intermittierenden Signales, das von dem Patienten (2) erfaßt wird, um Abfolgen von selbst-vergrößerenden Kontraktionen und Entspannungen einzuleiten, indem die Anzahl der Kontraktionen, die einen Hebekraftschwellwert nach ausreichender Entspannung, die durch eine ausreichend niedrige Hebekraft **gekennzeichnet** sind, gezählt werden.

11. Verfahren nach irgendeinem der Ansprüche 8 bis 10, **dadurch gekennzeichnet, daß** es weiterhin folgende Schritte enthält:
k) während der Patient (2) in der Position zum Messen der selbst-vergrößerenden Kraft der vorhergehenden Schritte ist, bei der eine normalen Fußsohlenanlage auf dem Fuß (3) stattfindet, Messen der maximalen nach vorne gerichteten Kraft des Oberschenkels (15) des Patienten (2) gegen die vordere Abstützung (14),
l) Wählen eines Schwellwertes der nach vorne gerichteten Anlagekraft unterhalb einer maximalen nach vorne gerichteten Anlagekraft des Oberschenkels (15),
m) Bestimmen der Ausdauer des Psoas-major-Muskels durch Messen der maximalen Zeit der Aufrechterhaltung einer nach vorne gerichteten Anlagekraft, die gleich oder größer des Schwellwertes der nach vorne gerichteten Anlagekraft ist, und/oder Zählen der Anzahl von abwechselnden Kontraktionen von Perioden der Entspannung und Erreichen des Schwellwertes der nach vorne gerichteten Anlagekraft.
